# EUROPEAN PATENT SPECIFICATION

(11) **EP 2 600 825 B1**
(45) Date of publication and mention of the grant of the patent: **12.12.2018**
(21) Application number: 10740486.5
(22) Date of filing: 02.08.2010
(51) Int. Cl.: A61K 8/42, A61Q 5/04, A61Q 5/12, A61Q 7/00

(54) **Method for curling hair**
Verfahren zum locekn von Haar
Procédé pour le bouclage de cheveux

(43) Date of publication of application: 12.06.2013
(73) Proprietor: AC PATENT HOLDING INC., Ventura, CA 93003 (US)
(72) Inventor: BRINKENHOFF, Michael, C., Ventura, CA 93002 (US)
(74) Representative: Studio Torta S.p.A.
(86) International application number: PCT/US2010/044170
(87) International publication number: WO 2012/018324

(56) References cited:
- WO-A1-2007/000642
- CA-A1- 2 735 825
- JP-A- 2003 321 442
- US-A- 5 886 035
- US-A- 5 985 920
- US-A1- 2003 199 590
- US-B1- 6 262 105
- US-B2- 7 351 404
- R.L.Rundle: "Drug that lengthens eyelashes sets off flutter", , 19 November 2007 (2007-11-19), XP002635657, Retrieved from the Internet: URL:http://online.wsj.com/article/SB119543 055372597359.html [retrieved on 2011-05-06]

## Description

### FIELD

Method for curling human hair including eyelashes.

### BACKGROUND

Certain therapeutic agents have been known to induce hair growth. One example is Minoxidil, 6-(1-piferidinyl)-2,4-pyrimidane-diamine 3-oxide (U.S. Patent Nos. 3,382,247 and 3,644,363). Minoxidil was originally prepared and sold for use as an antihypertensive. It was observed that, associated with the use of Minoxidil for this latter purpose, Minoxidil use also produced an increase in hair growth and thickness as reported in U.S. Patent Nos. 4,139,619 and 4,968,812. Today, Minoxidil is marketed under the trademark Rogaine® by Pfizer for the treatment of baldness on the scalp for men (alopecia androgenetica) and women. Another example is finasteride (Propecia®), marketed by Merck & Co. Finasteride was originally developed for benign prostatic hypertrophy, and was found to be effective in the treatment of alopecia androgenetica as reported in U.S. Patent No. 4,968,812.

Among the drugs introduced for lowering intraocular pressure are molecules of the family prostaglandin F2. The Upjohn Company identified a prostaglandin F2α analog, commonly known as Latanoprost and whose chemical name is isopropyl-(Z)-7[(1R, 2R, 3R, 5S)3,5-dihydroxy-2-[(3R)-3-hydroxy-5-phenylp-entyl]cyclopentyl]-5-heptenoate. Latanoprost is marketed by Pharmacia & Upjohn (currently a part of Pfizer) under the trademark Xalatan® for the reduction of elevated intraocular pressure in patients with glaucoma and ocular hypertension. The form is a Latanoprost optical solution of 0.005 % (50 µg/ml), and is applied by dropper directly onto the eye. One drop generally contains approximately 1.5 µg of Latanoprost.

In the course of its use for reduction of intraocular pressure, Latanoprost has been reported to cause, in some patients, an increasing pigmentation and growth of eyelashes. U.S. Patent No. 6,262,105 stated that the use of Latanoprost leads to increased length of lashes, increased numbers of lashes along the normal lash line, increased thickness and luster of lashes, increased auxiliary lash-like terminal hair in transitional areas adjacent to areas of normal lash growth, increased lash-like terminal hairs at the medial and lateral canthal area, increased pigmentation of the lashes, increased numbers, increased length, as well as increased luster, and thickness of fine hair on the skin of the adjacent lid, and increased perpendicular angulation of lashes and lash-like terminal hairs.

Alcon, Inc. ("Alcon") introduced a prostaglandin F2α analog, commonly known as Travoprost whose chemical name is isopropyl (z)-7-[(1R, 2R, 3R, 5S)-3,5-dihydroxy-2-[(1E, 3R)-3-hydroxy-4-[(α,α,α-trifluoro-m-tolyl)oxyl]-1-butenyl]cyclopentyl]-5-heptenoate as a glaucoma treatment. Alcon also sought patent protection for Travoprost for growing hair in U.S. Patent Application No. 2003/0199590.

Allergan, Inc. ("Allergan") introduced Bimatoprost whose chemical name is cyclopentane N-ethyl haptanamide-5-cis-2-(3α-hydrosy-5-phenyl-1-trans-pentenyl)-3,5-dihydroxy, [1α, 2α, 3α, 5α) for treating glaucoma. U.S. Patent No. 7,351,404 is directed at the use of this molecule and similar molecules for growing hair, including eyelashes. Allergan distinguishes the Bimatoprost molecule from other prostaglandins on the basis that Bimatoprost is a prostamide.

U.S. Patent Nos. 5,886,035 and 5,985,920 describe fluorine containing prostaglandin derivatives for use as a preventive or therapeutic medicine for an eye disease such as glaucoma or elevated intraocular pressure

CA2735825 discloses 15,15-difluoroprostaglandin derivatives for promoting growth of the hair. Aminocarbonyl and alkylaminocarbonyl derivatives are among those preferred.

### BRIEF DESCRIPTION OF THE DRAWINGS

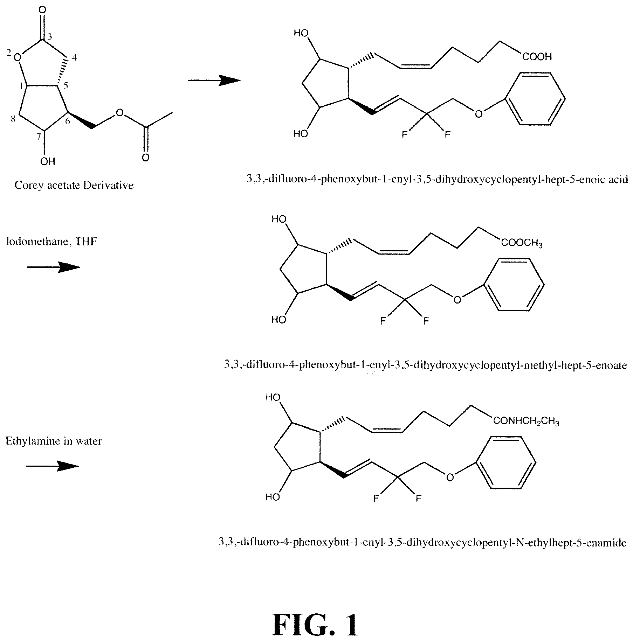

**Figure 1** shows a process flow from a Corey acetate derivative to a difluoro compound that includes an ethyl amide.

### DETAILED DESCRIPTION

Methods for curling hair are described. The location of the hair includes the scalp (e.g., scalp hair), eyelid (e.g., eyelashes), and brow (e.g., eyebrows) and any other portion of human skin where hair exists or may be desired.

In one embodiment, a composition for use in the method includes an effective hair enhancing amount of a compound represented by the following formula: 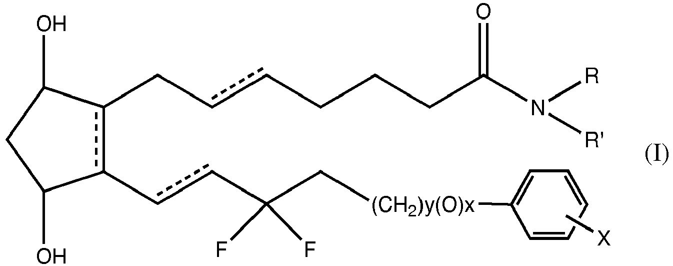 wherein the dashed bonds represent a single or double bond that can be in the cis or trans configuration; R and R' are individually selected from hydrogen, or a C₁-C₆ straight or branched chain alkyl (e.g., methyl, ethyl, isopropyl) that may be substituted (i.e., one or more of the hydrogen atoms that would be bound to a carbon atom in an alkyl group is replaced with another substituent (e.g., a serinol amide, -CH(CH₂OH)₂)); X is a halide (e.g., F, Cl, Br, I) a halide-containing group (e.g., CF₃), or hydrogen; either y=0 and x=1 or y=1 and x=0. The molecule may be characterized as a difluoro prostaglandin or prostaglandin-like compound because its backbone resembles a prostaglandin.

A difluoro compound may be in a molecular free form (a molecule) or an acceptable salt thereof (a compound). An acceptable salt is a dermatologically acceptable salt or a pharmaceutically acceptable salt. An example of a salt is a hydrohalide salt of the molecule such as a hydrochloride salt. In one embodiment, the resulting salt compound will have the proton from the hydrohalide bonded to the nitrogen of the molecule and be charge balanced by the halide. Throughout this description, unless specifically indicated a reference to a compound includes a molecule or a compound.

In another embodiment, the compound for use in the method has the following formula: 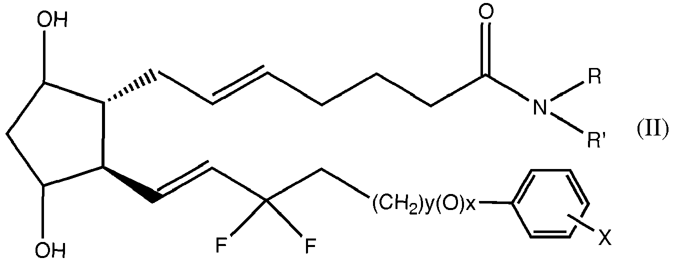 wherein R, R', X, y and x are defined above, or an acceptable salt thereof.

In a particular embodiment, the compound for use in the method is one of the following molecules or a salt thereof:

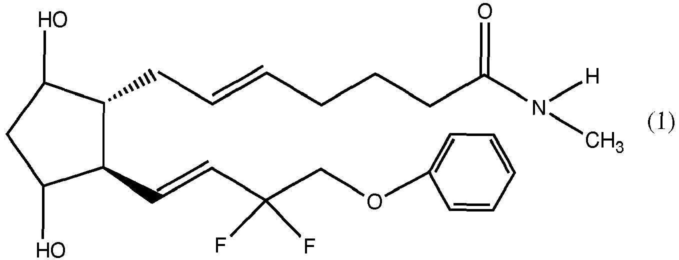

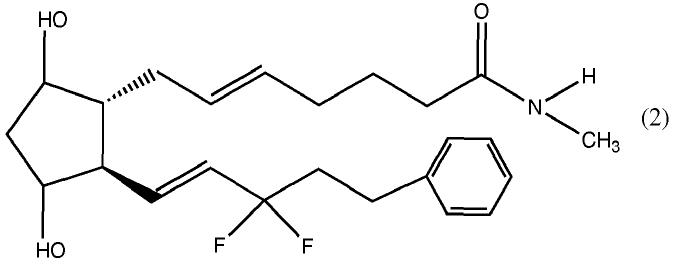

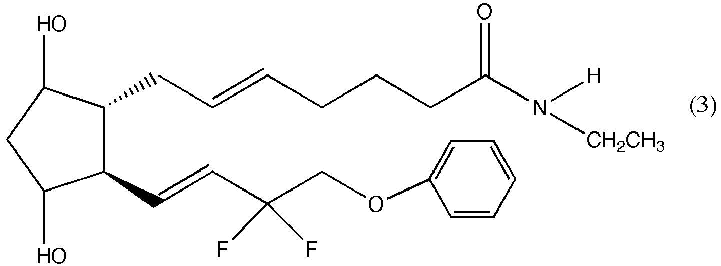

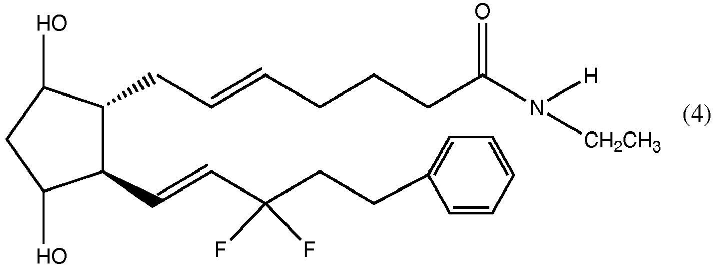

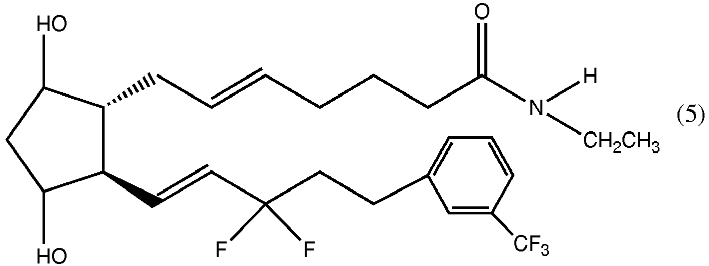

In one embodiment, one or more compounds described above is/are mixed with a dermatologically compatible vehicle or carrier to form a composition for use in the method. Suitable vehicles include, for example, aqueous solutions such as e.g., physiological salines, oil (e.g., castor oil), solutions, foams, creams or ointments. Suitable vehicles furthermore may contain dermatologically compatible preservatives such as, e.g., benzalkonium chloride, surfactants such as, e.g., polysorbate 80, liposomes or polymers, for example, methyl cellulose, polyvinyl alcohol, polyvinyl pyrrolidone and hyaluronic acid; these may be used for increasing the viscosity. Antimicrobials may also be added, such as, e.g., phenoxyethanol, chlorphenesin and/or parabens.

In another embodiment, a composition for use in the method may include a combination of difluoro prostaglandin or prostaglandin-like compounds described or a compound(s) and other hair treatment aids such as an antioxidant (e.g., Vitamin E), a vasodilator (e.g., minoxidil), an antimicrobial (e.g., benzoyl peroxide), or an anti-inflammatory (e.g., hydrocortisone, cyclooxygenase inhibitor, lipooxigenase inhibitor).

The composition may be topically applied to the dermis, meaning the surface of the skin, of, for example, a human in an area desired for enhancing hair such as direct application to existing hair or the dermis in an area desired for hair enhancing as opposed to an indirect application such as to the surface of the eye. Such location may include the scalp, eyelash area or eyelid or brow of a male or female. Application of the compound(s) or composition to a base of a hair or hairs, e.g., without necessarily actually touching the skin, may create a wicking action where the compound(s) or composition are drawn up the length of the hair or hairs toward the follicle(s). Repeated application for a sustained period of time (e.g., daily for several weeks or more (e.g., one month to several months)) may be desired.

Where hair is not present, the composition may be applied directly to the dermis where hair growth is desired. This may include the scalp where, for example, a density of existing hair has been reduced due to alopecia or other mechanism (e.g., hair loss as a side effect to chemotherapy treatment). In certain Asian cultures, an absence of pubic hair is considered bad luck and can be a serious problem, particularly in women. The composition may be applied to the pubic area to address this problem. In one embodiment, repeated daily topical application of a composition to an area of the dermis where hair growth is desired may continue until hair growth is effected and may continue periodically (daily, weekly) to maintain such growth. In one embodiment, such topical application to the dermis may, for example, utilize a delivery vehicle including the composition that is a foam or cream.

For application to eyelashes, a composition including at least one difluoro prostaglandin or prostaglandin-like compound as described above may be applied to the skin at the base of an eyelid adjacent to or where hair grows from the follicles (e.g., along the lash line). Alternatively or additionally, the composition may be applied to the eyelashes themselves. Repeated application for consecutive days, weeks or months (e.g., regular daily application for six months) of the composition to eyelashes or areas of eyelid dermis associated with eyelashes (e.g., the base of an eyelid containing follicles in which eyelashes grow) will condition and cosmetically enhance existing eyelashes such that the eyelashes appear longer, thicker and/or fuller. Such application will also stimulate or promote the growth of eyelashes.

In another embodiment, a kit for use in the method is disclosed. The kit may include the composition of at least one difluoro prostaglandin or prostaglandin-like compound as described above in a dermatologically compatible vehicle or carrier in a container. The container includes an amount of the composition for repeated application over a period, such as enough of the composition for daily application over a period of three to six months. The container may also include an applicator, such as a mascara brush, an eyeliner brush or other instrument for application of the composition to skin. In the case of a composition in the form of a foam, the container may be pressurized and include a nozzle to dispense a desired amount onto the hand of a user.

### EXAMPLE 1: Synthesis of 3,3-difluoro-4-phenoxybut-1-enyl-3,5-dihydroxycyclopentyl-hept-5-enoic acid

Starting from a Corey acetate derivative, an omega chain backbone, such as dimethyl 2-oxo-3-phenoxy-propyl phosphonate is added via an exchange reaction. The oxo group in the 2 position is preserved in the formed intermediate and then reduced with a fluorine compound to produce a difluoro precursor. Subsequently, the alpha chain is added at another site of the Corey acetate derivative yielding 3,3-difluoro-4-phenoxybut-1-enyl-3,5-dihydroxycyclopentyl-hept-5-enoic acid.

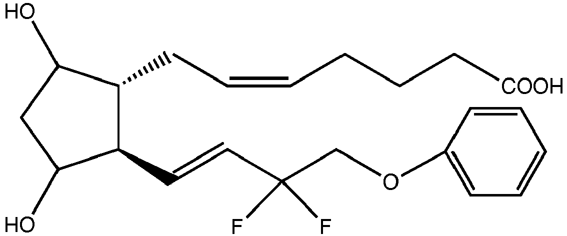

### EXAMPLE 2: Synthesis of 3,3-difluoro-4-phenoxybut-1-enyl-3,5-dihydroxycyclopentyl-menthylhept-5-enoate

To the 3,3-difluoro-4-phenoxybut-1-enyl-3,5-dihydroxycyclopentyl-hept-5-enoic acid is added iodomethane in a solution of tetrahydroturan (THF) to form 3,3-difluoro-4-phenoxybut-1-enyl-3,5-dihydroxycyclopentyl-methylhept-5-enoate.

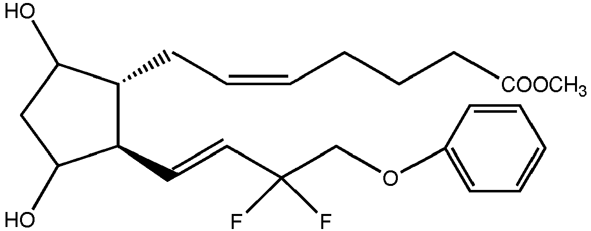

### EXAMPLE 3: Synthesis of 3,3-difluoro-4-phenoxybut-1-enyl-3,5-dihydroxycyclopentyl-N-ethylhept-5-enamide

To the 3,3-difluoro-4-phenoxybut-1-enyl-3,5-dihydroxycyclopentyl-methylhept-5-enoate is added ethylamine in water to form 3,3-difluoro-4-phenoxybut-1-enyl-3,5-dihydroxycyclopentyl-N-ethylhept-5-enamide.

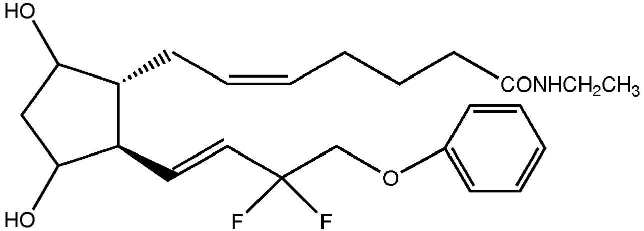

**Figure 1** shows a process flow from a Corey acetate derivative to the acid, to the ester and then to an ethyl amide (Compound 3). U.S. Patent No. 5,886,035 also describes a method of preparing difluoro-prostaglandin derivatives but does not describe the use of such derivatives in hair (eyelash) growth.

The following examples provide representative compositions including a difluoro compound such as Compound 3:

### EXAMPLE 4: Eyelash Composition

| **Ingredient** | | **Percentage** |
|---|---|---|
| 1. | Water (Aqua) | 97.791 |
| 2. | Sodium Chloride | 0.877 |
| 3. | Panthenol | 0.020 |
| 4. | Citric Acid | 0.030 |
| 5. | Phenoxyethanol | 0.300 |
| 6. | Chlorphenesin | 0.300 |
| 7. | Disodium Phosphate | 0.142 |
| 8. | Difluoro Compound | 0.024 |
| 9. | Alcohol | 0.216 |
| 10. | Cellulose Gum | 0.300 |

### EXAMPLE 5: Eyelash Composition

| **Ingredient** | | **Percentage** |
|---|---|---|
| 1. | USP Water | 94.519 |
| 2. | Sodium Chloride | 0.077 |
| 3. | Phosphoric Acid | 0.03 |
| 4. | Phenoxyethanol | 0.3 |
| 5. | Chlorphenesin | 0.3 |
| 6. | Disodium Phosphate | 0.142 |
| 7. | Cellulose Gum | 0.36 |
| 8. | Biotin | 0.5 |
| 9. | Glycerin | 0.6 |
| 10. | Swertia Japonica Extract | 0.045 |
| 11. | Saw Palmetto Extract | 0.064 |
| 12. | Camellia Sinensis (White Tea) Leaf Extract | 0.054 |
| 13. | Panax Ginseng | 0.075 |
| 14. | Octapeptide-2 | 0.93 |
| 15. | Biotinoyl Tripeptide-1 | 0.93 |
| 16. | Wheat Protein | 0.25 |
| 17. | Calendula | 0.8 |
| 18. | Difluoro Compound | 0.024 |

### EXAMPLE 6: Eyelash Composition

| **Ingredient** | | **Percentage** |
|---|---|---|
| 1. | USP Water | 96.031 |
| 2. | Actiphyte of Rosemary | 0.025 |
| 3. | Phosphoric Acid | 0.001 |
| 4. | Phenoxyethanol | 0.3 |
| 5. | Chlorphenesin | 0.3 |
| 6. | Disodium Phosphate | 0.985 |
| 7. | Cellulose Gum | 0.36 |
| 8. | Biotin | 0.24 |
| 9. | Panthenol | 0.25 |
| 10. | Actiphyte of Nettles | 0.1 |
| 11. | Actiphyte of Horsetail | 0.098 |
| 12. | Actiphyte of Psoralea Seed | 0.15 |
| 13. | Mulberry Root Extract | 0.07 |
| 14. | Actiphyte of Japanese Green Tea | 0.25 |
| 15. | Glycerin | 0.6 |
| 16. | Difluoro Compound | 0.024 |

### EXAMPLE 7: Scalp Composition

| **Ingredient** | | **Percentage** |
|---|---|---|
| 1. | Water | 95.00000 |
| 2. | Acrylates Copolymer | 1.50000 |
| 3. | Glycerin | 1.00000 |
| 4. | Phenoxyethanol | 0.80000 |
| 5. | Sodium Cocoyl Glutamate | 0.35000 |
| 6. | Chlorphenesin | 0.30000 |

| **Ingredient** | | **Percentage** |
|---|---|---|
| 7. | Polysorbate | 0.25000 |
| 8. | Difluoro Compound | 0.02400 |
| 9. | Disodium EDTA | 0.10000 |
| 10. | Fragrance | 0.09610 |
| 11. | Aminomethyl Propanol | 0.09000 |
| 12. | Panax Ginseng Root Extract | 0.08000 |
| 13. | Panthenol | 0.08000 |
| 14. | Ginko Bilola Leaf Extract | 0.05000 |
| 15. | Biotin | 0.05000 |
| 16. | Serenoa Serrulata Fruit Extract | 0.00640 |
| 17. | Swertia Japonica Extract | 0.00450 |
| | Hydrolyzed Wheat Protein | 0.00300 |
| 19. | Ethyl Alcohol | 0.21600 |

### EXAMPLE 8 (not according to the invention):

An experiment compared the activity of 3,3-difluoro-4-phenoxybut-1-enyl-3,5-dihydroxycyclopentyl-N-ethylhept-5-enamide (compound (3)) and 16-phenoxy PGF_{2_{α}} ethyl amide, molecules differing in subtituents at the C15 atom, with the latter having a hydrogen atom and a hydroxyl group (-OH) separately bonded to C15.

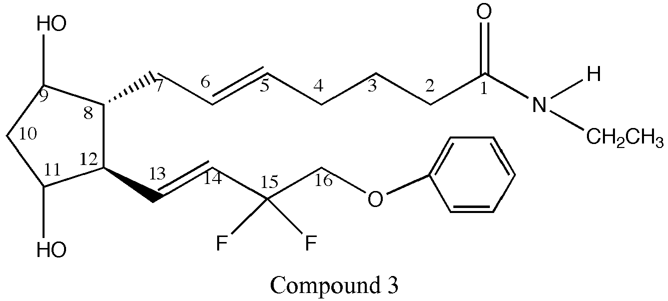

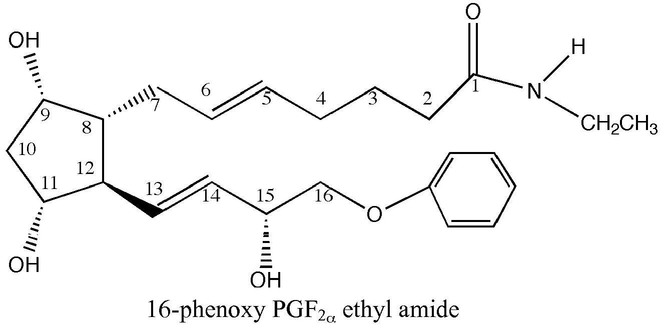

Test results indicate that there is a significant difference in the degree to which each of these molecules stimulates or promotes eyelash growth. Specifically, compound (3) causes eyelashes to grow noticeably within as few as 10-14 days in separate tests of daily applications of concentrations of 0.025%, 0.010% and 0.003%. At a daily application of a concentration of 0.025%, the growth was dramatic (described as a "pop"). Eyelash growth was not similarly apparent with the other molecule (16-phenoxy PGF_{2α} ethyl amide) at concentrations of 0.025%, 0.010% or 0.003% for several weeks or more. Even after as many as 12 weeks of application, the final effect of the application of the 16-phenoxy PGF_{2α} ethyl amide was significantly less than the effect of the application of compound (3) with regard to density (number of new lashes), thickness and length. An analysis of the effect of application of the compound (3) molecule indicated that the administration of the compound (3) molecule appeared to stimulate dormant eyelash follicles to begin generating new eyelashes and to cause existing eyelashes to grow at an accelerated rate.

The test results lead to a conclusion that the difluoride atoms at the C15 position significantly and uniquely improve the potential for enhancing hair, including stimulating or promoting hair growth. Without wishing to be bound by theory, it is believed that this observation is due to a unique biochemical pathway and mechanism of action, and that this pathway results in an unusually robust stimulation of a hair follicle.

It is postulated that there is a different and novel set of molecular receptor sites for a difluoro prostaglandin amide molecule having two fluorine atoms at the C15 position compared to a PGF_{2α} amide molecule with a hydroxyl group at the C15 position.

Methods of topical application of a composition to modify hair are described wherein the modification includes increased curl of hair on a subject or hair that is not biologically attached to or is not naturally a part of a subject (e.g., hair of a wig or extension) ("non-natural hair"). In another embodiment, the modification includes increased curl of the hair together with conditioning hair and/or cosmetically improving the appearance or beauty of the hair (e.g., increased look and feel of softness and silkiness). Thus, the compositions described may be applied to existing hair on the scalp or eyelashes or non-natural hair to increase the curl, the bulk (volume) and/or the look and feel of the hair. In one embodiment, a composition includes an effective amount of a compound represented by the following formula: 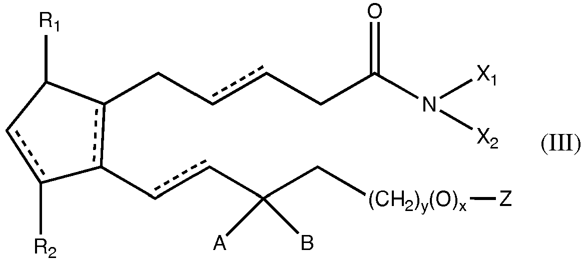
wherein the dashed bonds represent a single or double bond and when a bond in either chain is a double bond, cis or trans configuration is contemplated;
wherein A and B are individually selected from a hydrogen atom, a hydroxyl group (-OH) and a halogen atom (e.g., a fluorine atom), with the proviso that when one of A and B is a hydroxyl group, the other of A and B is a hydrogen atom and when one of A and B is a halogen atom, the other of A and B is a halogen atom or a hydrogen atom;
wherein Z is a cycloalkyl moiety having from three to seven carbon atoms, an aryl moiety including from four to ten carbon atoms or a heteroaryl moiety including three to nine carbon atoms and at least one heteroatom selected from nitrogen, oxygen and sulfur; and
wherein X₁ and X₂ are individually selected from a hydrogen atom, a C₁-C₆ straight or branched chain alkyl (e.g., methyl, ethyl, propyl) that may be substituted (e.g., a serinol amide, -CH(CH₂OH₂), 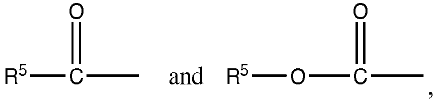 and
R⁵ is an alkyl moiety having from one to six carbon atoms;
wherein R₁ and R₂ are individually selected from an oxo group (=O), a hydroxyl group (-OH) or an ester group (-O(CO)R₆), and R₆ is a saturated or unsaturated acyclic hydrocarbon group having from 1 to about 20 carbon atoms, and -(CH₂)ₘR₇ wherein m is 0 or an integer of from 1 to 10, and R₇ is cycloalkyl group, having from three to seven carbon atoms, or an aryl or heteroaryl group, as defined above;
wherein y is 0 or 1, x is 0 or 1 and x and y are not both 1.

The compound may be in free form (a molecule) or an acceptable salt thereof (a compound) such as a cationic or anionic salt formed at the nitrogen atom of the molecule, in association with a carrier adapted for topical application to mammalian skin. Unless specified, reference to a compound will include a molecule or a compound.

In one embodiment, the compound is represented by the following formula: 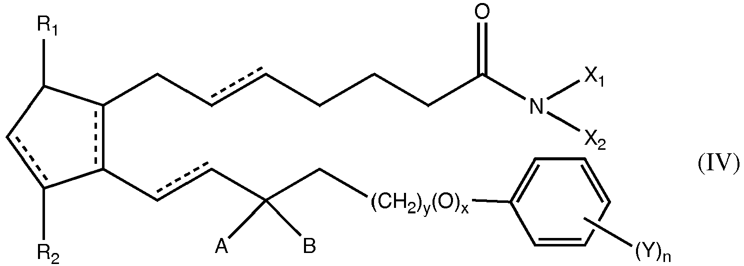 wherein A, B, X₁, X₂, x and y are as defined above and Y is selected from an alkyl group, a halogen atom (e.g., fluorine, chlorine), a nitro group, an amino group, a thiol group, a hydroxy group, an alkyloxy group, an alkylcarboxy group, a halo substituted alkyl wherein the alkyl includes from one to six carbon atoms, and n is 0 or an integer of from 1 to 3, or an acceptable salt thereof.

In another embodiment, the compound has the following formula: 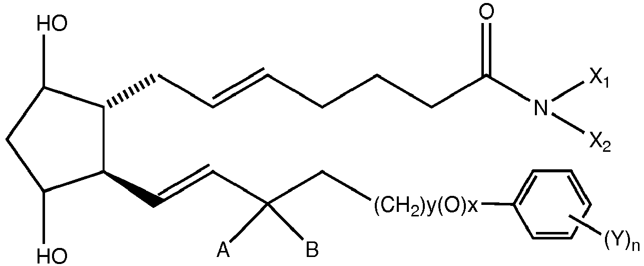 wherein A, B, X₁, X₂, x, y, Y and n are as defined above, or an acceptable salt thereof.

In a particular embodiment, the compound is selected from any of compounds (1)-(5) above or a molecule having one of the following formulas or a salt thereof:

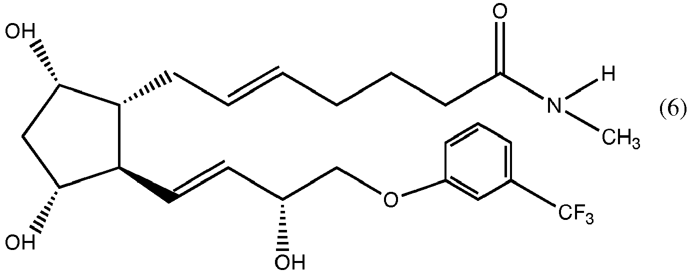

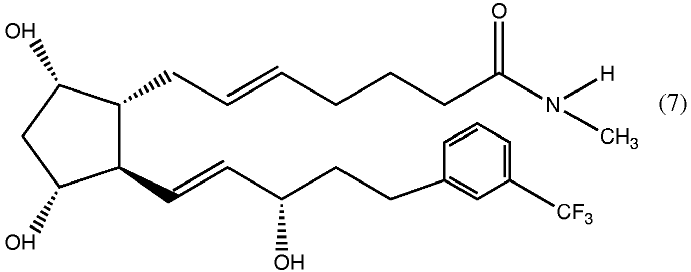

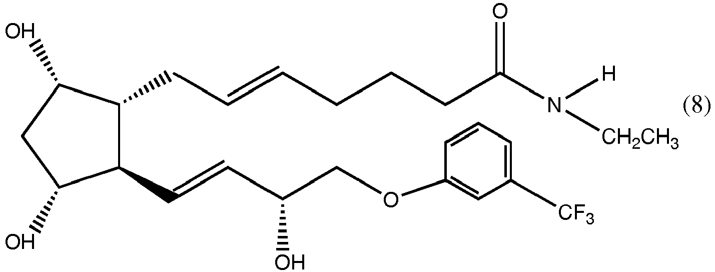

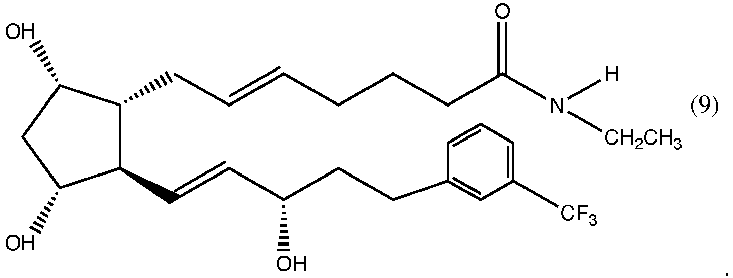

Compounds (6)-(9) may be prepared using techniques known in the art including, for example, techniques described in U.S. Patent Nos. 5,001,153; 5,422,368; 5,510,383; and 5,607,978.

In one embodiment, the one or more compounds described above (including compounds (1)-(9) is/are mixed with a dermatologically compatible vehicle or carrier. Suitable vehicles include, for example, aqueous solutions such as e.g., physiological salines, oil (e.g., castor oil), water solutions, water/alcohol solutions or ointments. Suitable vehicles furthermore may contain dermatologically compatible preservatives such as e.g., benzalkonium chloride, surfactants like e.g., polysorbate 80, liposomes or polymers, for example, methyl cellulose, polyvinyl alcohol, polyvinyl pyrrolidone and hyaluronic acid; these may be used for increasing the viscosity.

In one embodiment, dermatological compositions for topical treatment for inducing or stimulating modification of existing hair including increased curl which include an effective hair modifying amount of one or more compounds as defined above and a dermatologically compatible carrier are also disclosed wherein the modification includes increased curl of hair or increased curl. Effective amounts of the compound may be determined by one of ordinary skill in the art but will vary depending on the compound employed, frequency of application and desired result, and the compound will generally range from about 0.0000001 to about 50%, by weight, of the dermatological composition, preferably from about 0.001 to about 50%, by weight, of total dermatological composition, more preferably from about 0.1 to about 30%, by weight of the composition. In another embodiment, a composition may include a combination of compounds described or a compound(s) and other hair treatment aids such as an antioxidant (e.g., Vitamin E), a vasodilator (e.g., minoxidil), an antimicrobial (e.g., benzoyl peroxide), or an anti-inflammatory (e.g., hydrocortisone, cyclooxygenase inhibitor, lipooxigenase inhibitor).

The composition may be topically applied to existing hair and retained on the hair. Such location may include the scalp or eyelash area of a male or female. Repeated application for a sustained period of time (e.g., daily for several weeks or more (e.g., one month to several months)) may be desired. The composition may be beneficial to persons that naturally have straight scalp hair to aid in the curl of such hair. Evidence of the effectiveness of a composition of compound (3) (dechloro-dihydroxy-difluoro-ethylcloprostenate) and, alternatively, of compound (8) (trifluoromethyl dechloroethyl prostenolamide) in application to generally straight Asian hair indicates a profound curling effect.

In one embodiment, the composition is suitable for modifying eyelashes. To modify eyelashes, a composition would be provided to a subject with instructions on use (e.g., by instructions on or with a kit, the kit including the composition and the instructions). The subject would be instructed to apply the composition to eyelash hair. In addition to modifying the eyelashes (e.g., increasing the curl and/or look and feel), it is anticipated that application to a hair follicle will increase the thickness (e.g., diameter) of the follicle.

In one embodiment, the composition may be topically applied to hair on the scalp. For example, a subject interested in establishing a curl to a portion of the subject's scalp hair or improving a natural curl, would be instructed (e.g., by instructions on or with a kit) to topically apply the composition to the desired portion of the hair and to leave the composition on the hair. To improve or sustain the curl over a period of time (e.g., days, weeks), the subject would be instructed to repeat the application for a period ranging from days to weeks.

In another embodiment, the applications described above may be used to modify non-natural hair (e.g., hair not biologically attached to or is not naturally a part of a subject). Examples of such hair include, but are not limited to, wigs and hair extensions.

In another embodiment, a kit for use in the method is disclosed. The kit may include the composition of at least one compound having the formula III or IV as described above in a dermatologically compatible vehicle or carrier in a container. The container includes an amount of the composition for repeated application over a period, such as enough of the composition for daily application over a period of six months. The container may also include an applicator, such as a mascara brush or other instrument for application of the composition to skin. In the case of a composition in the form of a foam, the container may be pressurized and include a nozzle to dispense a desired amount onto the hand of a user.

### EXAMPLE 9 (not according to the invention)

The following example used trifluoromethyl dechloroethyl prostenolamide (TDP) as a compound. TDP has the following formula:

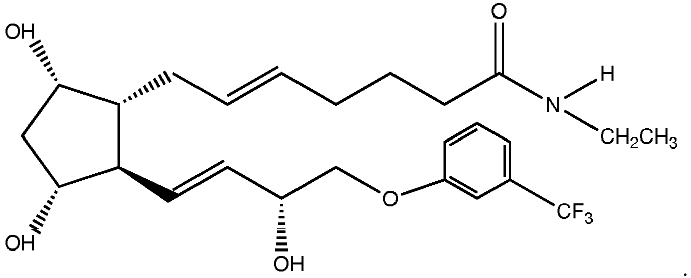

A solution of TDP was prepared including 97.6 percent water, 0.24 percent TDP and 2.16 percent ethyl alcohol. Samples of human hair separated from a subject were treated with water alone while others were treated with the TDP solution. The treatment involved dipping the dry hair samples into the water and TDP solutions to one minute. Unexpectedly, there appeared a definite increase in curl in each of the hair samples when treated with the TDP solution. The hair treated with water alone did not show similar curl. The results are illustrated in **Figure 1**. In addition, the hair treated with the TDP solution had a noticeable and palpable increase in the look and feel of softness and silkiness compared to the hair treated with the water alone.

## Claims

1. A method comprising:
directly applying to hair an effective hair curling amount of a composition to increase a curl of the hair, the composition comprising a compound of the formula: 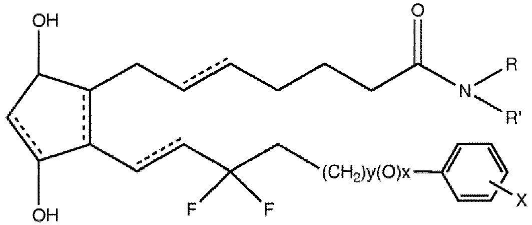 wherein the dashed bonds represent a single or double bond, R and R' are individually selected from the group consisting of hydrogen, a C₁-C₆ straight or branched chain alkyl, X is selected from the group consisting of a halide, a halide containing group or a hydrogen, y is 0 or 1, x is 0 or 1 and x and y are not both 1, or a salt thereof.

2. The method of claim 1, wherein the hair comprises eyelashes.

3. The method of claim 1, wherein the compound has the formula:

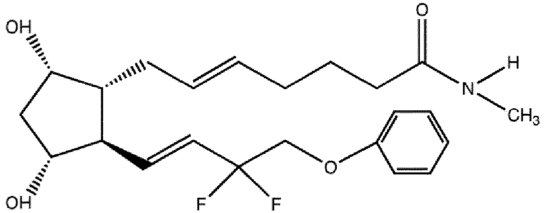

4. The method of claim 1, wherein the compound has the formula:

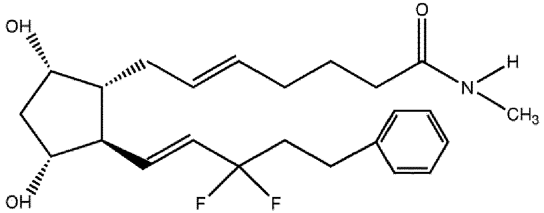

5. The method of claim 1, wherein the compound has the formula:

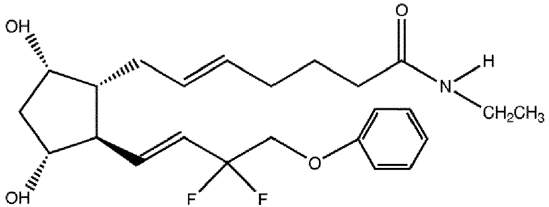

6. The method of claim 1, wherein the compound has the formula:

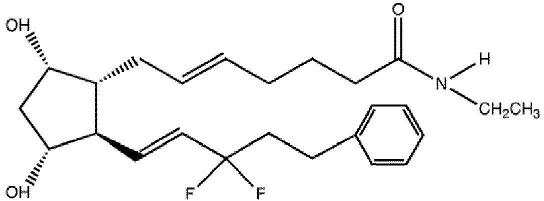

7. The method of claim 1, wherein the composition further comprises a dermatologically acceptable carrier.

8. A method comprising:
directly applying to hair on a dermis an effective hair curling amount of a composition to increase a curl of the hair, the composition comprising a compound of the formula: 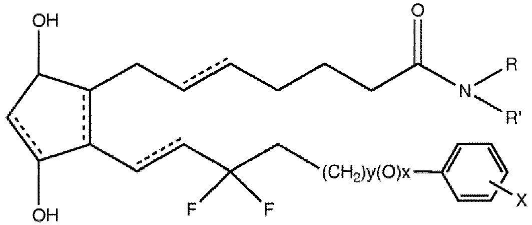 wherein the dashed bonds represent a single or double bond, R and R' are individually selected from the group consisting of hydrogen, a C₁-C₆ straight or branched chain alkyl, X is selected from the group consisting of a halide, a halide containing group or a hydrogen, y is 0 or 1, x is 0 or 1 and x and y are not both 1, or a salt thereof.

9. The method of claim 8, wherein the compound has the formula of one of the following:

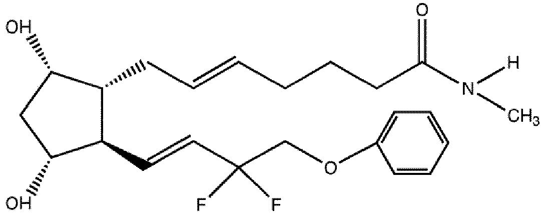

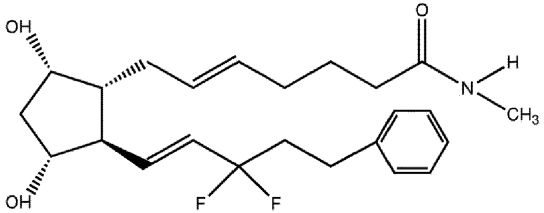

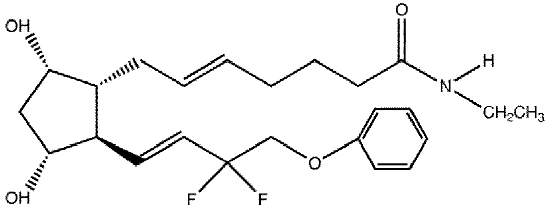

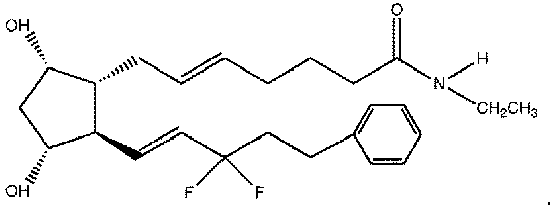

## Patentansprüche

1. Verfahren, umfassend:
direktes Aufbringen auf Haar einer wirksamen Haarkräuselmenge einer Zusammensetzung, um das Locken bzw. Kräuseln des Haars zu steigern, wobei die Zusammensetzung eine Verbindung der Formel umfaßt: 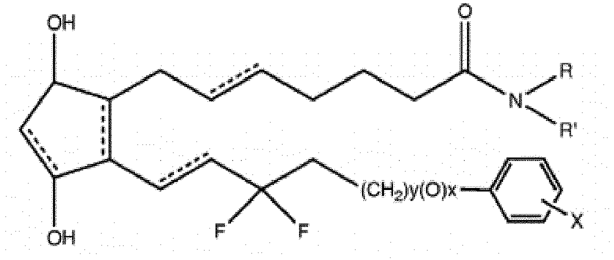 wobei die gestrichelten Bindungen eine Einfach- oder Doppelbindung darstellen, R und R' unabhängig voneinander aus der Gruppe, bestehend aus Wasserstoff, einem C₁-C₆ geradkettigem oder verzweigtkettigem Alkyl ausgewählt sind, X aus der Gruppe, bestehend aus einem Halogenid, einer halogenhaltigen Gruppe oder einem Wasserstoff ausgewählt ist, y 0 oder 1 ist, x 0 oder 1 ist und x und y beide nicht 1 sind, oder ein Salz davon.

2. Verfahren gemäß Anspruch 1, wobei das Haar Augenlider umfaßt.

3. Verfahren gemäß Anspruch 1, wobei die Verbindung die Formel aufweist:

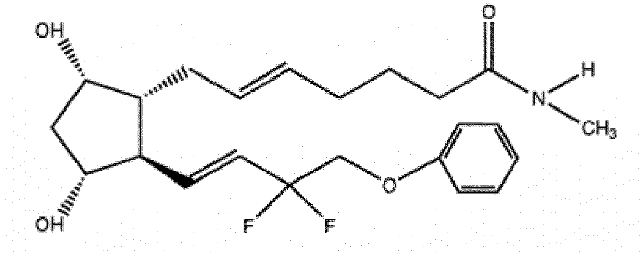

4. Verfahren gemäß Anspruch 1, wobei die Verbindung die Formel aufweist

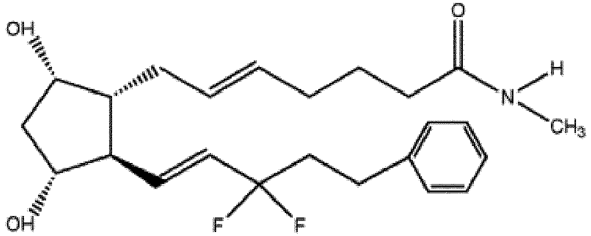

5. Verfahren gemäß Anspruch 1, wobei die Verbindung die Formel aufweist:

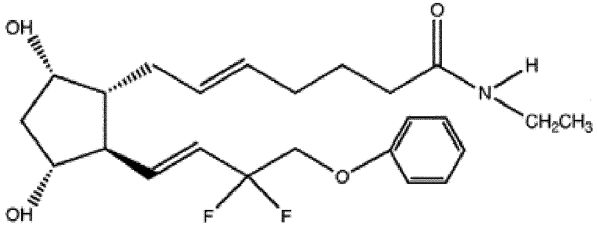

6. Verfahren gemäß Anspruch 1, wobei die Verbindung die Formel aufweist:

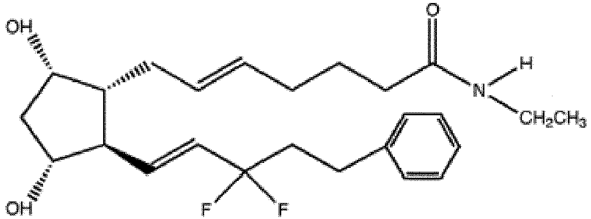

7. Verfahren gemäß Anspruch 1, wobei die Zusammensetzung weiter einen dermatologisch verträglichen Träger umfaßt.

8. Verfahren, umfassend:
direktes Aufbringen einer wirksamen Haarkräuselmenge von einer Zusammensetzung auf Haar auf einer Dermis, um ein Locken bzw. Kräuseln des Haars zu steigern, wobei die Zusammensetzung eine Verbindung der Formel umfaßt: 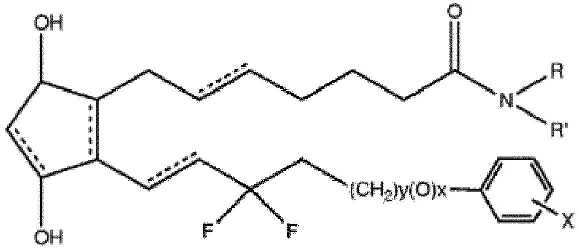 wobei die gestrichelten Bindungen eine Einfach- oder Doppelbindung darstellen, R und R' unabhängig voneinander aus der Gruppe, bestehend aus Wasserstoff, einem C₁-C₆ geradkettigem oder verzweigtkettigem Alkyl ausgewählt sind, X aus der Gruppe, bestehend aus einem Halogenid, einer halogenhaltigen Gruppe oder einem Wasserstoff ausgewählt ist, y 0 oder 1 ist, x 0 oder 1 ist und x und y beide nicht 1 sind, oder ein Salz davon.

9. Verfahren nach Anspruch 8, wobei die Verbindung die Formel von einer der folgenden aufweist:

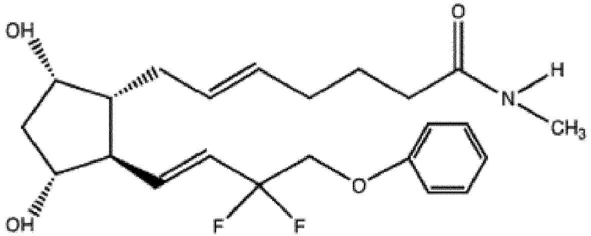

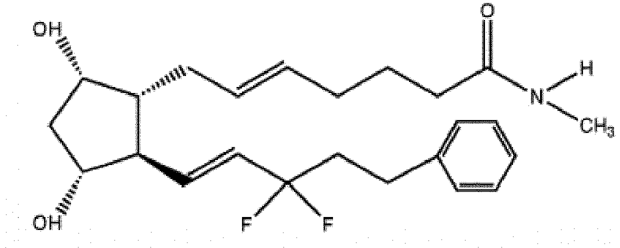

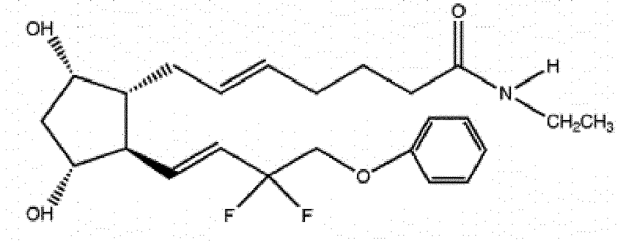

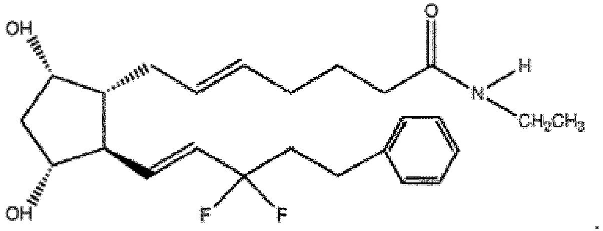

## Revendications

1. Procédé comprenant :
l'application directement aux cheveux d'une quantité d'une composition efficace pour boucler les cheveux pour augmenter le bouclage des cheveux, la composition comprenant un composé de formule : 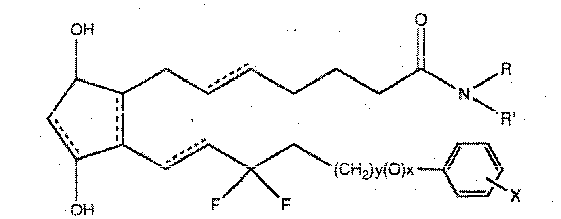 dans laquelle les liaisons en pointillés représentent une liaison simple ou double, R et R' sont individuellement choisis dans le groupe constitué par un hydrogène, un alkyle en C₁-C₆ à chaîne linéaire ou ramifiée, X est choisi dans le groupe constitué par un halogénure, un groupe contenant un halogénure, ou un hydrogène, y est 0 ou 1, x est 0 ou 1 et x et y ne sont pas tous les deux 1, ou un sel de celui-ci.

2. Procédé selon la revendication 1, dans lequel les cheveux comprennent les cils.

3. Procédé selon la revendication 1, dans lequel le composé a la formule :

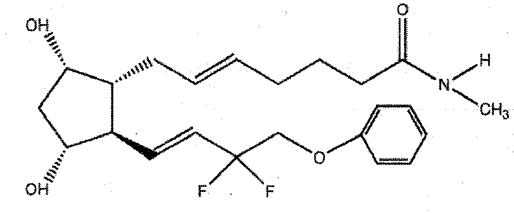

4. Procédé selon la revendication 1, dans lequel le composé a la formule :

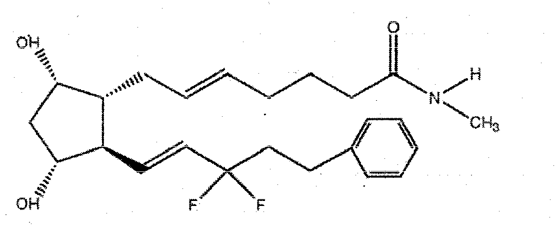

5. Procédé selon la revendication 1, dans lequel le composé a la formule :

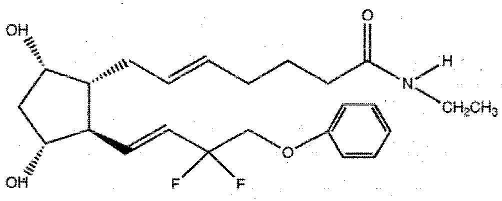

6. Procédé selon la revendication 1, dans lequel le composé a la formule :

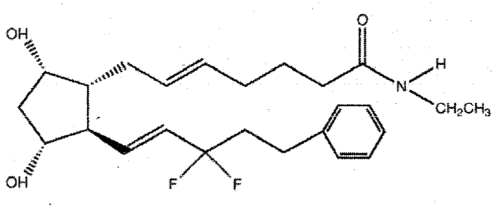

7. Procédé selon la revendication 1, dans lequel la composition comprend en outre un véhicule dermatologiquement acceptable.

8. Procédé comprenant :
l'application directement aux cheveux sur un derme d'une quantité d'une composition efficace pour boucler les cheveux pour augmenter le bouclage des cheveux, la composition comprenant un composé de formule : 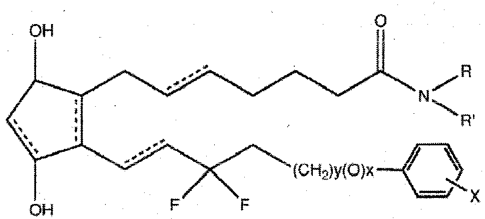 dans laquelle les liaisons en pointillés représentent une liaison simple ou double, R et R' sont individuellement choisis dans le groupe constitué par un hydrogène, un alkyle en C₁-C₆ à chaîne linéaire ou ramifiée, X est choisi dans le groupe constitué par un halogénure, un groupe contenant un halogénure, ou un hydrogène, y est 0 ou 1, x est 0 ou 1 et x et y ne sont pas tous les deux 1, ou un sel de celui-ci.

9. Procédé selon la revendication 8, dans lequel le composé a la formule parmi l'une des suivantes :
